# EUROPEAN PATENT APPLICATION

(11) **EP 2 116 270 A1**
(43) Date of publication of application: **11.11.2009**
(21) Application number: 09251055.1
(22) Date of filing: 07.04.2009
(51) Int. Cl.: A61M 16/08

(54) **Gas supply system**

(30) Priority: 06.05.2008 US 115842
(71) Applicant: Davis, Tom, Orient, OH 43146 (US); Davis, Nancy, Orient, OH 43146 (US)
(72) Inventor: Davis, Tom, Orient, OH 43146 (US); Davis, Nancy, Orient, OH 43146 (US)
(74) Representative: Jehan, Robert

(57) **Abstract**

A gas supply system (100) is disclosed. The gas supply system (100) includes a housing (102), a spool (104), a gas tube (106), at least one opening (108-110), a retract mechanism and a holding member (112). The spool (104) is configured within the housing (102). The gas tube (106) is retractably would on the spool (104) and includes a first end portion and a second end portion of the gas tube (106) to extend out of the housing. The retract mechanism is configured in the housing (102) and is capable of retracting a portion of the gas tube (106) extended out of the housing (102). The holding member (112) extends from the housing and enables the gas supply system (100) to be removably mounted on a supporting member.

## Description

### FIELD OF THE INVENTION

The present invention relates generally to delivery of therapeutic gases such as oxygen, nitrous oxide and the like to patients, and, more particularly, to a gas supply system capable of delivering a gas or a therapeutic gas from a gas supply to a nasal cannula connected to a patient's nose.

### BACKGROUND OF THE INVENTION

During surgeries and other medical treatments, patient often require a supply of therapeutic gases, for example, oxygen, nitrous oxide, and the like. A source of the therapeutic gases may include an air canister gas supply system, an air supply cylinder and the like. The therapeutic gases may be supplied to the patient from the source using a gas tube and a nasal cannula. More specifically, one end of the gas tube is connected to the source and the other end to the nasal canula. Further, the nasal cannula is used to administer the therapeutic gases into the patient through his/her nose.

Typically, the gas tube connected to the source of the therapeutic gas may have a sufficient length, thereby enabling the patient to move within an area of a room. However, due to the movement of the patient inside the room, the gas tube may drag along the floor of the room. Consequently, such a long length of the gas tube may enhance the probability of the patient tripping or falling on the gas tube and hurting himself/herself.

Moreover, when the gas tube is dragged along a dirty floor of the room due to the patient's movement, the gas tube may get soiled. Accordingly, the patient using such a soiled gas tube may catch infection. Further, the gas tube lying on the floor or dragged along the room may affect aesthetic appeal of the room and may provide an unorganized environment to the patient.

### SUMMARY OF THE INVENTION

The present invention seeks to provide an improved gas supply system.

According to an aspect of the present invention, there is provided a gas supply system as specified in claim 1.

The preferred embodiment provides a gas supply system that allows a patient to move freely and conveniently inside a room while being administered a therapeutic gas from the gas supply system. Moreover, the gas supply system can prevent a gas tube from lying on a floor of the room and being dragged along the floor.

The gas supply system can keep a gas tube clean, thereby reducing chances of causing infection to a patient.

The system can also enable a user to preserve the aesthetics of the room, thereby providing an organized environment to the patient.

In one embodiment, a gas supply system is provided which is capable of delivering gas from a gas supply to a nasal cannula and capable of being removably mounted on a supporting member. The gas supply system includes a housing, a spool, a gas tube, at least one opening, a retract mechanism and a holding member. The spool is configured within the housing and rotatably coupled to the housing. The gas tube is retractably wound on the spool. The gas tube has a first end portion and a second end portion. The first end portion of the gas tube is capable of being connected to the gas supply. The second end portion of the gas tube is capable of being connected to the nasal cannula. The at least one opening is configured on the housing for enabling at least one of the first end portion and the second end portion of the gas tube to extend out of the housing. The retract mechanism is configured in the housing and operatively coupled to the spool. The retract mechanism is capable of retracting a portion of the gas tube extended out of the housing. The holding member extends from the housing and enables the gas supply system to be removably mounted on the supporting member.

Preferably, the gas supply system includes a retaining mechanism slidably engaged with the portion of the gas tube extended out of the housing. The retaining mechanism is capable of retaining the extended portion of the gas tube outside the housing.

These together with other aspects of the present invention, along with the various features of novelty that characterize the present invention, are pointed out with particularity in the claims annexed hereto and form a part of this present invention. For a better understanding of the present invention, its operating advantages, and the specific objects attained by its uses, reference should be made to the accompanying drawings and descriptive matter in which there are illustrated exemplary embodiments of the present invention.

### BRIEF DESCRIPTION OF THE DRAWINGS

The advantages and features of the present invention will become better understood with reference to the following detailed description and claims taken in conjunction with the accompanying drawings, in which:

FIG. 1A illustrates a gas supply system used for delivery of a gas from a gas supply to a nasal cannula, in accordance with an embodiment of the present invention;

FIG. 1B illustrates an exploded view of the gas supply system of FIG. 1A, in accordance with an embodiment of the present invention; and

FIG. 2 illustrates an environment in which the gas supply system of FIGS. 1A and 1B is being utilized by a patient for administering a gas thereto, in accordance with an embodiment of the present invention.

Like reference numerals refer to like parts throughout the description of several views of the drawings.

### DETAILED DESCRIPTION OF THE INVENTION

The exemplary embodiments described herein detail for illustrative purposes are subject to many variations in structure and design. It should be emphasized, however, that the present invention is not limited to a particular gas supply system, as shown and described. It is understood that various omissions and substitutions of equivalents are contemplated as circumstances may suggest or render expedient, but these are intended to cover the application or implementation without departing from the spirit or scope of the claims of the present invention.

The terms "first," "second," and the like, herein do not denote any order, quantity, or importance, but rather are used to distinguish one element from another, and the terms "a" and "an" herein do not denote a limitation of quantity, but rather denote the presence of at least one of the referenced item.

The present invention provides a gas supply system. The gas supply system is adapted to be used for delivery of a gas from a gas supply, for example, an air supply cylinder, to a nasal cannula. The gas supply system enables the patient to move conveniently and freely from one place to another inside a room while being administered a gas from the disclosed gas supply system. Moreover, the gas supply system prevents the tube from lying on a floor of the room and being dragged thereof along the floor.

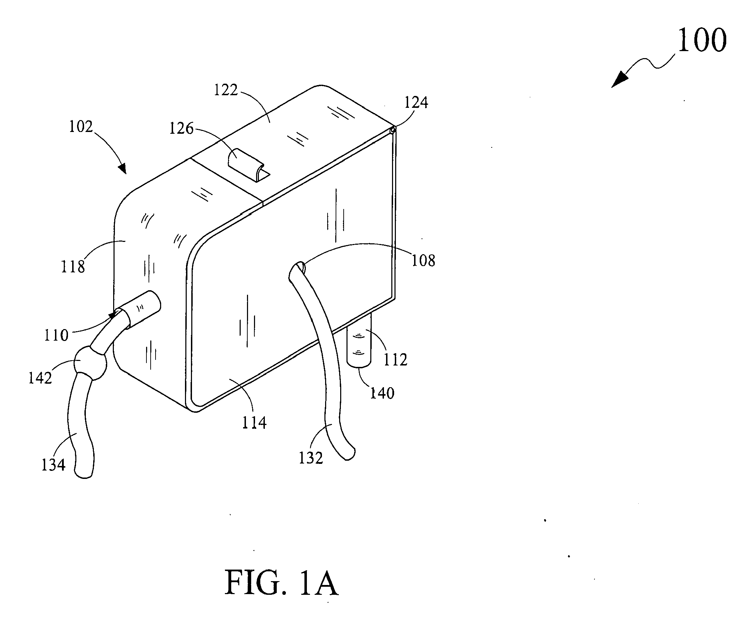

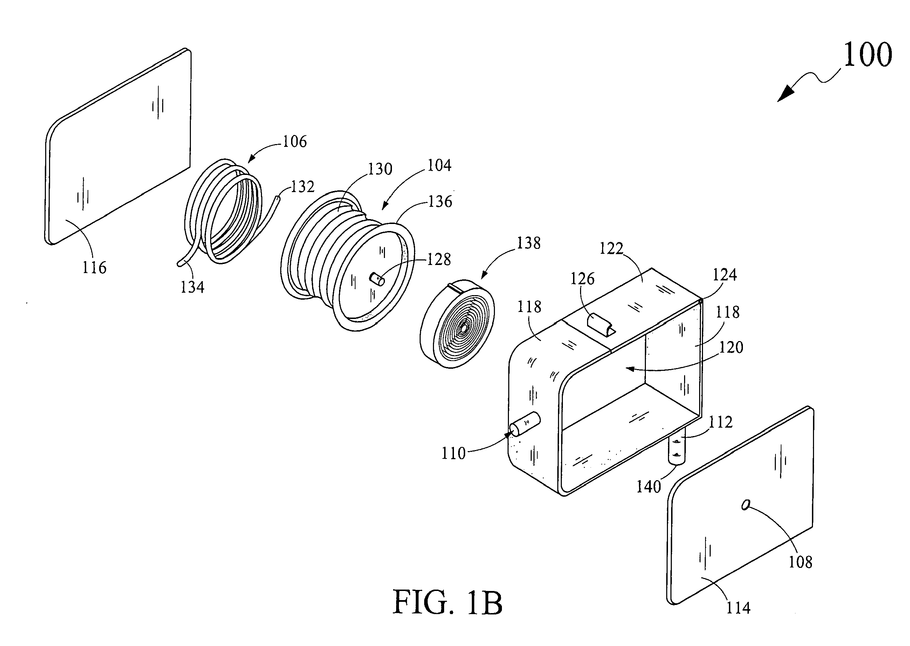

Referring to FIG. 1A and FIG. 1B, a gas supply system 100 is illustrated. More specifically, FIG. 1A illustrates an assembled view of the gas supply system 100, in accordance with an embodiment of the present invention; and FIG. 1B illustrates an exploded view of the gas supply system 100, in accordance with an embodiment of the present invention. The gas supply system 100 (hereinafter referred to as 'system 100') includes a housing 102, a spool 104, a gas tube 106, at least one opening for example, a first opening 108 and a second opening 110, a retract mechanism (not shown) and a holding member 112.

In an embodiment of the present invention, the housing 102 may be configured to form a cuboidal structure as shown in FIGS. 1A and 1B. More specifically, the housing 102 may include a rectangular first wall, for example, a first wall 114; a rectangular second wall, for example, a second wall 116 substantially parallel to the first wall 114; and at least one peripheral wall 118 (hereinafter referred to as 'peripheral wall 118'). The peripheral wall 118 extends between the first wall 114 and the second wall 116 to form a cavity 120 therebetween.

For the purpose of this description the housing is shown and described to assume a cuboidal structure. However, it will be evident to a person skilled in the art that apart from the cuboidal structure, the housing 102 may be configured to have other structures such as a spherical structure, a cylindrical structure, a polygonal structure, and the like.

In an embodiment of the present invention, the peripheral wall 118 is at least partially openable for enabling an access to the cavity 120 of the housing 102. For example, the system 100 may include a removable portion 122 configured in the peripheral wall 118. The removable portion 122 may be detached completely or partially from the peripheral wall 118 for enabling an access to the cavity 120 of the housing 102. In an embodiment of the present invention, the removable portion 122 may have an end operatively coupled to the peripheral wall 118 of the housing 102 through a coupling arrangement 124. The coupling arrangement 124 may be any coupling arrangement known in the art, for example, a hinged coupling arrangement, a pivotal coupling arrangement, and the like. The removable portion 122 of the peripheral wall 118 may include a holder 126 configured thereon for facilitating removal of the removable portion 122.

Further, without limiting the scope of the present invention, the housing 102 may be made of a transparent material. More specifically, the housing 102 may be composed of a clear plastic material that renders the housing 102 transparent. However, it will be apparent to a person skilled in the art that the housing 102 may be composed of any other materials known in the art, such as, a plastic material, a metallic material, and the like.

The spool 104 of the system 100 is configured within the housing 102 and rotatably coupled to the housing 102. More specifically, the spool 104 is configured within the cavity 120 of the housing 102. Accordingly, the spool 104 may rotatably move within the cavity 120 of the housing 102. The spool 104 may be coupled to the housing 102 using any coupling mechanisms known in the art.

In an embodiment of the present invention, the spool 104 may be disposed on a spindle 128. The spindle 128 may have a pair of ends rotatably coupled with the first wall 114 and the second wall 116 of the housing 102. In such an embodiment, the rotation of the spindle 128 enables the spool 104 to rotate within the cavity 120 of the housing 102. Further, such a configuration of the spool 104 as set forth herein is for the purpose of illustration and description only. It will be evident to a person skilled in the art that the spool 104 may have any other configurations that may enable the spool 104 to be configured within the housing 102 of the system 100.

In another embodiment of the present invention, the spool 104 is removably coupled to the housing 102. More specifically, the spool 104 is removably coupled to the first wall 114 and the second wall 116 of the housing 102. Accordingly, the spool 104 may be removed from the housing 102 by at least partially opening the peripheral wall 118. Thereafter, the removed spool 104 may be replaced with any other spools. In the present embodiment, the spool 104 may be removed from the housing 102 by opening the removable portion 122 of the housing 102 using the holder 126.

The spool 104 is capable of holding the gas tube 106 thereon. More specifically, the gas tube 106 is retractably wound on the spool 104. In an embodiment of the present invention, the spool 104 may include a plurality of grooves, such as the plurality of grooves 130 (as shown in FIG. 1B), hereinafter referred to as grooves 130 configured thereon. More specifically, the grooves 130 may act as individual compartments capable of receiving the gas tube 106 wound on the spool 104. The grooves 130 facilitate proper alignment of the gas tube 106 wound on the spool 104. Moreover, the grooves 130 prevent compression of the gas tube 106 due to overlapping thereof upon being wound on the spool 104. In one embodiment of the present invention, the number of grooves 130 in a spool, for example, the spool 104 may vary with the length of the gas tube 106.

As disclosed herein, the gas tube 106 supplies gas present in the gas supply to the patient through the nasal cannula. Examples of the gas may include, but are not limited to a therapeutic gas such as oxygen, nitrous oxide, and the like. The gas tube 106 has a first end portion 132 and a second end portion 134. The first end portion 132 of the gas tube 106 is capable of being connected to a gas supply (not shown) and the second end portion 134 of the gas tube 106 is capable of being connected to a nasal cannula (not shown). In one embodiment of the present invention, the first end portion 132 is fixedly attached to a periphery 136 of the spool 104. Further, the second end portion 134 unwinds the gas tube 106 from the spool 104 upon rotation of the spool 104. Moreover, such a configuration of the gas tube 106 and the spool 104 as set forth herein is for the purpose of illustration and description only. It will be evident to a person skilled in the art that any other configuration of the gas tube 106 and the spool 104, known in the art can be included in the gas supply system 100.

Further, in an embodiment of the present invention, the gas tube 106 may be made of a plastic material. However, it will be apparent to a person skilled in the art that the gas tube 106 may be made of any other material known in the art such as a rubber material, and on the like. Furthermore, in an embodiment of the present invention, the gas tube 106 may be provided in a variety of colors such as fluorescent neon or any other color.

The housing 102 further includes the at least one opening configured thereon for enabling at least one of the first end portion 132 and the second end portion 134 of the gas tube 106 to extend out of the housing 102. In an embodiment of the present invention, the at least one opening may be a single opening configured on the housing 102 for enabling the first end portion 132 and the second end portion 134 of the gas tube 106 to extend out of the housing 102.

In another embodiment, the at least one opening may include more than one opening. For example, the at least one opening may include the first opening 108 and the second opening 110 configured in the first wall 114 and the peripheral wall 118 respectively, of the housing 102 as shown in FIGS. 1A and 1B. The first opening 108 enables the first end portion 132 of the gas tube 106 to extend out from the housing 102. The first end portion 132 of the gas tube 106 extended out from the housing 102 is connected to the gas supply. Further, the second opening 110 enables the second end portion 134 of the gas tube 106 to extend out of the housing 102. The extended second end portion 134 of the gas tube 106 is connected to the nasal cannula. It will be apparent to a person skilled in the art that the first opening 108 and the second opening 110 of the system 100 may be configured in any part of the housing 102 such as the first wall 114, the second wall 116 and the peripheral wall 118.

The retract mechanism is configured in the housing 102 and operatively coupled to the spool 104. The retract mechanism is capable of retracting a portion of the gas tube 106 extended out of the housing 102. More particularly, the retract mechanism enables the extended portion of the gas tube 106 to retract into the cavity 120 of the housing 102 through the second opening 110. The extended portion of the gas tube 106 retractably winds on the spool 104. In an embodiment of the present invention, the retract mechanism may include a protrusion (not shown) configured on the first wall 114 of the housing 102 such that manual operation of the protrusion may facilitate rotation of the spool 104. Upon operating the protrusion, the spool 104 may rotate, thereby retracting the extended portion of the gas tube 106 into the housing 102.

In an embodiment of the present invention, the retract mechanism may be a spring mechanism 138 coupled with the spool 104 and the spindle 128. The spring mechanism 138 enables the spool 104 to retractably rotate such that the extended portion of the gas tube 106 retracts into the cavity 120 of the housing 102. More specifically, when a patient moves away from the system 100, the gas tube 106 extends out through the second opening 110 of the housing 102 by forcing the spool 104 to rotate and the spring mechanism 138 to expand. Further, when the patient moves closer to the system 100, the spring mechanism 138 contracts and forces the spool 104 to rotate in a direction so as to retract the gas tube 106 into the housing 102. Moreover, it will be apparent to a person skilled in the art that the retract mechanism may include any other mechanisms known in the art for retracting the extended portion of the gas tube 106.

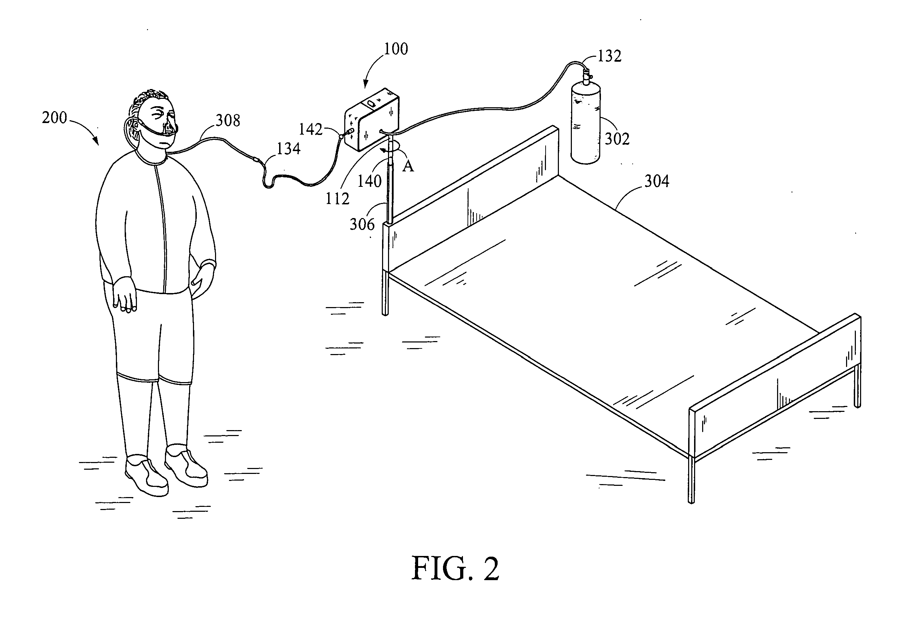

The holding member 112 of the system 100 has a bottom end portion 140, a swivel mechanism (not shown) operably coupled to the bottom end portion 140 of the holding member 112. The holding member 112 extends from the peripheral wall 118 of the housing 102. The holding member 112 enables the system 100 to be removably mounted on a supporting member, for example, a wheel chair, a bed, and the like. The swivel mechanism enables the system 100 to rotatably move on the supporting member upon being mounted thereon. It will be apparent to a person skilled in the art that the swivel mechanism may be a mechanism known in the art used for enabling the system 100 to execute a rotational motion when the system 100 is mounted on the supporting member. The holding member 112 may be received by a swivel head configured on the supporting member for removably mounting the system 100 on the supporting member. The swivel head of the supporting member will be explained further in conjunction with FIG. 2.

The system 100 further includes a retaining mechanism 142 slidably engaged with the extended portion of the gas tube 106. The retaining mechanism 142 is capable of retaining the extended portion of the gas tube 106 outside the housing 102. For instance, the gas tube 106 may be extended out of the housing 102 through the second opening 110 by a user. Thereafter, the user may slide the retaining mechanism 142 to a position on the gas tube 106 such that the extended portion of the gas tube 106 is restrained from retracting into the housing 102. The retaining mechanism 142 may possess a bead like structure as shown in FIG. 1A. However, it will be apparent to a person skilled in the art that the retaining mechanism 142 may possess any other structures known in the art.

FIG. 2 illustrates a perspective view of the system 100 being utilized by a patient 200 while being administered a gas, for example, oxygen from a gas supply 302, in accordance with an embodiment of the present invention. The system 100 may be mounted on a supporting member, for example, a bed 304 by using the holding member 112. The bed 304 may have a receiving member 306 that may receive the bottom end portion 140 of the holding member 112 to securely mount the system 100 on the bed 304. In one embodiment of the present invention, the holding member 112 may be connected to the swivel head configured on the supporting member using a connector. The connector may be a bushing connector, a swivel connector or any other connector known in the art. The connector enables the system 100 to rotate freely while being utilized by a patient, for example, the patient 200. Moreover, the connector prevents entangling of the gas tube 106. It will be apparent to a person skilled in the art that the connector may be internally or externally configured at the bottom end portion 140 of the holding member 112.

The swivel mechanism and connector configured at the bottom end portion 140 of the holding member 112 enables the system 100 to execute a rotational motion as shown by an arrow 'A'. Further, the arrow 'A' has been depicted showing rotation in one direction for illustration purpose only. However, the system 100 is capable of being rotated in other direction as well. In an embodiment of the present invention, the receiving member 306 may have the swivel head (not shown) capable of coupling with the bottom end portion 140 of the holding member 112.

The system 100 is connected to the gas supply 302 and a nasal cannula 308 is connected to a nose of the patient 200. As explained in conjunction with FIGS. 1A and 1B, the first end portion 132 of the gas tube 106 may be connected to the gas supply 302, and the second end portion 134 connected to the nasal cannula 308 respectively. More specifically, the first end portion 132 of the gas tube 106 extends out from the first opening 108 of the system 100 and engages with the gas supply 302; while the second end portion 134 of the gas tube 106 extends out from the second opening 110 of the system 100 and engages with the nasal cannula 308.

In use, when the patient 200 moves away from the bed 304, a desired length of the gas tube 106 extends out through the second opening 110 of the system 100, thereby enabling the patient 200 to conveniently move around. As described herein, only the desired length of the gas tube 106 extends out from the system 100. Accordingly, the gas tube 106 may not lie on the floor of the room or drag along the floor, thereby avoiding tripping and falling of the patient 200. Moreover, the patient 200 may use the retaining mechanism 142 to retain the extended portion of the gas tube 106 outside the housing 102. When the patient 200 moves closer to the system 100, the retaining mechanism 142 may be disengaged and the extended portion i.e. the desired length of the gas tube 106 may be manually retracted back into the system 100 using the retract mechanism such as the protrusion. In an embodiment of the present invention, the extended portion of the gas tube 106 may be automatically retracted by the retract mechanism such as the spring mechanism 138 of the system 100.

In an embodiment of the present invention, the system 100 may be about 4 inches in width and about 6 inches in length, thereby providing a compact structure thereto. Moreover, the gas tube 106 configured within the system 100 may have a length of about 25 feet. In another embodiment of the present invention, the system 100 may be about 4 inches in width and about 10 inches in length. Further, the gas tube 106 may have a length of about 50 feet. Moreover, the system 100 may have weight ranging from about 1 pound to about 2 pounds. Additionally, the system 100 may be provided in a variety of sizes having dimensions other than the dimensions mentioned in the above embodiments.

The gas supply system 100 explained in conjunction with FIGS. 1A, 1B and 2 may be advantageously used for delivery of a gas from a gas supply to a nasal cannula. The disclosed gas supply system may include a gas tube retractably extending therefrom for enabling the patient to move freely and conveniently while being administered gas from the gas supply system. Further, as the gas tube of the gas supply system is retractable, the gas tube may not lie or drag along a floor of a room, thereby reducing a probability of tripping and falling of the patient over the gas tube. Furthermore, the retractable feature of the disclosed gas supply system provides an organized environment or room for the patient. Moreover, the gas supply system prevents soiling of the gas tube by avoiding a contact of the gas tube with the floor, thereby reducing the chances of causing infections to the patient.

The foregoing descriptions of specific embodiments of the present invention have been presented for purposes of illustration and description. They are not intended to be exhaustive or to limit the present invention to the precise forms disclosed, and obviously many modifications and variations are possible in light of the above teaching. The embodiments were chosen and described in order to best explain the principles of the present invention and its practical application, and to thereby enable others skilled in the art to best utilize the present invention and various embodiments with various modifications as are suited to the particular use contemplated. It is understood that various omissions and substitutions of equivalents are contemplated as circumstances may suggest or render expedient, but such omissions and substitutions are intended to cover the application or implementation without departing from the scope of the claims.

The disclosures in United States patent application No. 12/115842, from which this application claims priority, and in the abstract accompanying this application are incorporated herein by reference.

## Claims

1. A gas supply system for delivery of gas from a gas supply to a nasal cannula and capable of being removably mounted on a supporting member, the gas supply system including:
a housing;
a spool configured within the housing and rotatably coupled to the housing:
a gas tube retractably wound on the spool, the gas tube including a first end portion and a second end portion, the first end portion of the gas tube capable of being connected to the gas supply, and the second end portion of the gas tube capable of being connected to the nasal cannula;
at least one opening configured on the housing for enabling at least one of the first end portion and the second end portion of the gas tube to extend out of the housing;
a retract mechanism configured in the housing and operatively coupled to the spool, the retract mechanism capable of retracting a portion of the gas tube extended out of the housing; and
a holding member extending from the housing, the holding member enabling the gas supply system to be removably mounted on the supporting member.

2. The gas supply system of claim 1, wherein the housing includes:
a first wall;
a second wall substantially parallel to the first wall; and
at least one peripheral wall extending between the first wall, and the second wall forming a cavity therebetween.

3. The gas supply system of claim 2, wherein the at least one peripheral wall is at least partially openable for enabling an access to the cavity of the housing.

4. The gas supply system of claim 1, 2 or 3, wherein the housing is made of a transparent material.

5. The gas supply system of any preceding claim, wherein the housing is at least partially made of a plastic material.

6. The gas supply system of any preceding claim, wherein the spool is removably coupled to the housing.

7. The gas supply system of any preceding claim, wherein the gas tube is made of a plastic material.

8. The gas supply system of any preceding claim, including a swivel mechanism operably coupled to an end of the holding member, the swivel mechanism capable of enabling the gas supply system to rotatably move upon being mounted on the supporting member.

9. The gas supply system of any preceding claim, including a retaining mechanism slidably engaged with the portion of the gas tube extended out of the housing, the retaining mechanism capable of retaining the extended portion of the gas tube outside the housing.
